# EUROPÄISCHE PATENTSCHRIFT

(11) **EP 0 500 605 B1**
(45) Veröffentlichungstag und Bekanntmachung des Hinweises auf die Patenterteilung: **03.05.1995**
(21) Anmeldenummer: 90916115.0
(22) Anmeldetag: 02.11.1990
(51) Int. Cl.: A61K 7/32, A61K 7/48

(54) **DESODORIERENDE KOSMETISCHE MITTEL**
COSMETIC DEODORANTS
DEODORANTS COSMETIQUES

(30) Priorität: 16.11.1989 DE 3938140
(43) Veröffentlichungstag der Anmeldung: 02.09.1992
(73) Patentinhaber: Beiersdorf Aktiengesellschaft, 20245 Hamburg (DE)
(72) Erfinder: SCHMUCKER, Robert, D-2000 Hamburg 61 (DE); SAUERMANN, Gerhard, D-2351 Wiemersdorf (DE); EIGENER, Ulrich, D-2000 Hamburg 65 (DE); ENGEL, Walter, D-2080 Pinneberg (DE)
(86) Internationale Anmeldenummer: DE9000837
(87) Internationale Veröffentlichungsnummer: WO9107164

(56) Entgegenhaltungen:
- EP-A- 0 181 578
- EP-A- 0 342 486
- DE-A- 2 359 225
- Seifen - öle - Fette - Wachse, 97. Jg., Nr. 16, 5. August 1971, Ing. Chem. H.Bartl: "Kosmetik-Rohstoffe", Antibiotika als Deodorantien", Seite 556-557,siehe Abschnitte 5,6

## Beschreibung

Die vorliegende Erfindung betrifft kosmetische Desodorantien. Solche Formulierungen dienen dazu, Körpergeruch zu beseitigen, der entsteht, wenn der an sich geruchlose frische Schweiß durch Mikroorganismen zersetzt wird. Den handelsüblichen kosmetischen Desodorantien liegen unterschiedliche Wirkprinzipien zugrunde.

Durch Adstringentien - vorwiegend Aluminiumsalze wie Aluminiumhydroxychlorid - kann die Entstehung des Schweißes unterbunden werden. Abgesehen von der Denaturierung der Hautproteine greifen die dafür verwendeten Stoffe aber drastisch in den Wärmehaushalt der Achselregion ein und sollten allenfalls in Ausnahmefällen angewandt werden.

Durch antimikrobielle Stoffe kann die Bakterienflora auf der Haut reduziert werden. Dabei sollten im Idealfalle nur die Geruch verursachenden Mikroorganismen vernichtet werden. In der Praxis hat sich aber herausgestellt, daß die gesamte Mikroflora der Haut in gleichem Maße geschädigt werden. Gelegentlich werden sogar die Mikroorganismen, die keinen Geruch verursachen, stärker geschädigt.

Schließlich kann Körpergeruch auch durch Duftstoffe überdeckt werden, die klassische Methode, die aber am wenigsten den ästhetischen Bedürfnissen des Verbrauchers gerecht wird, da die Mischung aus Körpergeruch und Parfümduft eher unangenehm riecht.

Desodorantien sollen folgende Bedingungen erfüllen:
1) Die biologischen Vorgänge der Haut dürfen nicht beeinträchtigt werden
2) Die Desodorantien sollen keinen ausgeprägten Eigengeruch besitzen
3) Sie müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in handelsübliche kosmetische Formulierungen einarbeiten lassen.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays Intimreinigungsmittel usw.

Aufgabe der vorliegenden Erfindung war es, kosmetische Desodorantien zu entwickeln, die die Nachteile des Standes der Technik nicht aufweisen. Insbesondere sollten die Desodorantien die Mikroflora der Haut weitgehend schonen, die Mikroorganismen aber, die für den Körpergeruch verantwortlich sind, selektiv reduzieren.

Es wurde gefunden, und darin liegt die Lösung der Aufgabe, daß die Verwendung eines wirksamen Gehaltes von Lantibiotika in kosmetischen Desodorantien, wobei die Lantibiotika sowohl einzeln als auch im Gemisch vorliegen können, den Nachteilen des Standes der Technik abhelfen.

Lantibiotika sind seit vielen Jahren bekannt. Es sind Polypeptide, die von Mikroorganismen synthetisiert werden und sich durch das Vorhandensein von Lanthionin in der Peptidsequenz auszeichnen.

Die Lanthionine haben folgende Strukturen:
HOOC-CH(NH₂)-CH₂-S-CH₂-CH(NH₂)-COOH
andere Schreibweise: 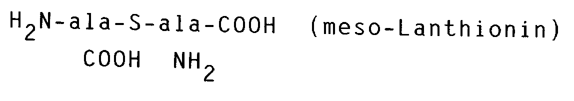
sowie
HOOC-CH(NH₂)-CH₂-S-CH(CH₃)-CH(NH₂)-COOH
andere Schreibweise: 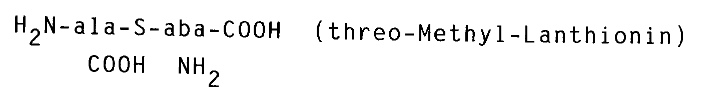
Mit diesen Lanthioninen werden in den Lantibiotika Ringstrukturen gebildet.

Beispiele für Lantibiotika sind Nisin, Epidermin, Subtilin, Cinramycin, Duramycin, Ancovenin, Gallidermin, Pep 5.

Die vorgenannten Stoffe sind an sich bekannt und können unter den Registraturnummerm der Chemical Abstracts ermittelt werden:

| | |
|---|---|
| Nisin | 1414-45-5 |
| Epidermin | 99165-17-0 |
| Subtilin | 1393-38-0 |
| Pep 5 | 110655-58-8 |
| Duramycin | 1391-36-2 |
| Ancovenin | 88201-41-6 |
| Gallidermin | 117978-77-5 |

Nisin beispielsweise ist ein Peptid aus 34 Aminosäuren, welches von Streptococcus lactis synthetisiert wird. Es wirkt vorwiegend gegen Micrococcen und coryneforme Bakterien. In manchen Ländern ist es ein als Lebensmittelkonservierungsstoff zugelassenes Antibiotikum (nicht in Deutschland).

Nisin besitzt folgende Aminosäurensequenz (Primärstruktur): 
S

H2N-ile-dhb-ala-ile-dha-leu-ala-aba-pro-gly-ala-lys-

S

-aba-gly-ala-leu-met-gly-ala-asn-

S

S

-met-lys-aba-ala-aba-ala-his-ala-

S

-ser-ile-his-val-dha-lys-COOH
wobei
- dhb =: Dehydrobutyrin
- dha =: Dehydroalanin
- aba =: Aminobuttersäure
E.Gross, J.L.Morell , "The Structure of Nisin" , J.Amer.Chem.Soc. 93, Ss. 4634-4637 (1971).

Der Wirkungsmechanismus von Nisin und den mit Nisin verwandten und im wesentlichen wirkungsgleichen Lantibiotika kann wie folgt gedeutet werden: Durch Freisetzung von Autolysinen werden Zellwände zerstört. Da zudem in der Cytoplasmamembran Kanäle gebildet werden, können niedermolekulare Zellbestandteile ausdiffundieren, wodurch die (prokaryotische) Zelle vernichtet wird.

Die Richtigkeit dieser Deutung ist indes für die Erfindung ohne Belang. Es soll nur der Versuch unternommen werden, die mikrobiologischen Vorgänge zu erläutern.

Eukaryotische Zellen, also Hautzellen, Pilze usw, sind gegen Nisin und andere Lantibiotika resistent.

Lantibiotika sind für den Warmblüter praktisch ungiftig. Die LD50 für Nisin beispielsweise ist größer als 7 g/kg (ermittelt für Ratten und Katzen).

In "Seifen-Öle-Fette-Wachse", 97. Jg., Nr. 16, 5.August 1981, Ing. Chem. H. Bartl: "Kosmetik-Rohstoffe, Antibiotika als Deodorantien" S. 556 - 557, werden Untersuchungen auf Tauglichkeit von Antibiotika als Deodorantien beschrieben. Zitiert sind Penicillin, Streptomycin, Neomycin, also Breitspektrenantibiotika, die imstande sind, auch die unschädlich Mikroflora zu ruinieren. Diese aber soll erfindungsgemäß geschont werden. In der Fußnote (loc.cit., S.556 unten) wird dann auch die Empfehlung ausgesprochen, auf Verwendung von Antibiotika solle bei der Herstellung kosmetischer Präparate verzichtet werden. Darüberhinaus gehören die zitierten Antibiotika Strukturklassen an, die mit Lantibiotika nichts gemein haben.

In EP-A-0 342 486 wird die Verwendung von Gallidermin in Medikamenten beschrieben, welche gegen bestimmte Krankheitserreger, beispielsweise bei Hautkrankheiten, aktiv sind. Anspruch 9 dieser Schrift betrifft zwar verbal ein kosmetisches Mittel, enthaltend Gallidermin, also einen Vertreter der Lantibiotika. Die Offenbarung dieser Schrift indes läßt keine Rückschlüsse auf eine Verwendung in desodorierenden Kosmetika zu. Angeführt wird einzig und allein die Verwendung von Gallidermin als Mittel gegen Akne, eine den Betroffenen stark psychisch belastende Krankheit, welche nur im allerweitesten Sinne als kosmetisches Problem aufgefaßt werden kann.

Es war erstaunlich und für den Fachmann nicht vorhersehbar, daß kosmetische Zusammensetzungen mit einem Gehalt an Lantibiotika gemäß der vorliegenden Erfindung
- bei der Lagerung stabil sein würden
- eine genügend hohe Halbwertszeit auf der Haut besitzen würden
- zur Anwendung als Kosmetikum geeignet sein würden
- selektiv gegen gerucherzeugende Mikroorganismen wirksam sein würden
- die symbiontische Mikroflora der Haut schonen würden.

Insbesondere war erstaunlich, daß die erfindungsgemäßen Zusammensetzungen nicht nur für kosmetische Zwecke geeignet sind, sondern überdies wirkungsvoller und schonender sind als die Zusammensetzungen des Standes der Technik.

Es hat sich herausgestellt, daß sich Lantibiotika erfolgreich in alle gewöhnlichen Formulierungstypen von Desodorantien einarbeiten lassen.

Vorzugsweise liegen die Lantibiotika in Konzentrationen von 0,1 - 10000 ppm vor. Besonders bevorzugt liegen die Lantibiotika in Konzentrationen von 0,1 - 750 ppm, ganz besonders bevorzugt in Konzentrationen von 0,5 - 400 ppm, vor. Die Konzentrationsangaben beziehen sich jeweils auf den Gehalt an reinem Wirkstoff und auf das Gesamtgewicht der Zusammensetzung.

Als besonders vorteilhaft haben sich Formulierungen mit einem wirksamen Gehalt an Nisin, Epidermin und/oder Gallidermin herausgestellt. Aber auch die anderen genannten Lantibiotika sind gut für die erfindungsgemäße Verwendung geeignet.

Es ist möglich und gegebenenfalls vorteilhaft, ein Gemisch an Lantibiotika als wirksames Prinzip der erfindungsgemäßen Zusammensetzungen einzusetzen.

Es ist vorteilhaft, die erfindungsgemäßen Zusammensetzungen auf einen pH-Bereich von 2,5 - 6,5 abzupuffern. Besonders günstig ist es, den pH-Wert in einem Bereich von 3,5 - 4,8 zu wählen.

Es kann ferner von Vorteil sein, den Zusammensetzungen Zusatzstoffe einzuverleiben, die die Stabilität der Lantibiotika erhöhen, beispielsweise Konservierungsstoffe, Antioxidantien, andere Proteine, Photostabilisatoren usw.

Ansonsten sind die üblichen Maßregeln für das Zusammenstellen von kosmetischen Formulierungen zu beachten, die dem Fachmann geläufig sind.

Die Lantibiotika können auf einfache Weise in die erfindungsgemäßen Zusammensetzungen eingearbeitet werden. Bevorzugt werden sie in gelöster Form (z.B. als wäßrige, alkoholische oder alkoholisch-wäßrige Lösung) den übrigen Bestandteilen der Formulierungen zugesetzt. Ganz besonders vorteilhaft aber ist es, Lantibiotika in sogenannte Pudersprays einzuarbeiten. Vorteilhaft ist es auch, Zusatzstoffe zu vermeiden, welche die natürliche Mikroflora schädigen können, da die Lantibiotika für sich selektiv die geruchserzeugenden Mikroorganismen reduzieren

Sollen Lantibiotika in Pudersprays eingearbeitet werden, so können die Suspensionsgrundlagen dafür vorteilhaft gewählt werden aus der Gruppe Aerosil, Magnesiumcarbonat, Magnesiumoxid, Kieselgur, Kaolin, Talkum, modifizierte Stärke, Metallseifen (z.B. Zinkstearat), Titandioxid, Aluminiumoxid, Calciumcarbonat, Zinkoxid, Seidenpulver, Nylonpulver, Polyethylenpulver und verwandten Stoffen.

Die folgenden Beispiele dienen dazu, die Erfindung zu beschreiben, ohne daß beabsichtigt ist, die Erfindung auf diese Beispiele zu beschränken.

### Beispiel 1

### Pumpspray

| | |
|---|---|
| Nisin (reine Substanz) | 0,025 g |
| Ethanol pharm. (96 %) | 354,875 g |
| Parfüm, Farbstoff | nach Belieben |
| Wasser | auf 1 000,000 g |

### Beispiel 2

### Pumpspray

| | |
|---|---|
| Epidermin | 0,010 g |
| Ethanol pharm. (96 %) | 354,875 g |
| Parfüm, Farbstoff | nach Belieben |
| Wasser | auf 1 000,000 g |

### Beispiel 3

### Deo-Roller (Roll-on)

| | |
|---|---|
| Nisin | 0,150 g |
| Hydroxyethylcellulose | 5,000 g |
| Propylenglycol | 5,000 g |
| Ethanol pharm. (96 %) | 355,850 g |
| Parfüm, Farbstoff | nach Belieben |
| Wasser | auf 1 000,000 g |

### Beispiel 4

### Deo-Roller (Roll-on)

| | |
|---|---|
| Epidermin | 0,120 g |
| Hydroxyethylcellulose | 5,000 g |
| Propylenglycol | 5,000 g |
| Ethanol pharm. (96 %) | 355,850 g |
| Parfüm, Farbstoff | nach Belieben |
| Wasser | auf 1 000,000 g |

### Beispiel 5

### Spray

| | |
|---|---|
| Nisin | 0,200 g |
| Ethanol pharm. (96 %) | 150,000 g |
| Propylenglycol | 50,000 g |
| Dimethylether | 300,000 g |
| Parfüm | nach Belieben |
| Wasser | auf 1 000,000 g |

### Beispiel 6

### Spray

| | |
|---|---|
| Epidermin | 0,150 g |
| Ethanol pharm. (96 %) | 150,000 g |
| Propylenglycol | 50,000 g |
| Dimethylether | 300,000 g |
| Parfüm | nach Belieben |
| Wasser | auf 1 000,000 g |

### Beispiel 7

### Spray

| | |
|---|---|
| Nisin | 0,180 g |
| Ethanol pharm. (96 %) | 497,160 g |
| 2-Octyldodecanol | 2,660 g |
| Parfüm | nach Belieben |
| Dimethylether | auf 1 000,000 g |

### Beispiel 8

### Spray

| | |
|---|---|
| Epidermin | 0,150 g |
| Ethanol pharm. (96 %) | 497,160 g |
| 2-Octyldodecanol | 2,660 g |
| Parfüm | nach Belieben |
| Dimethylether | auf 1 000,000 g |

### Beispiel 9

| | |
|---|---|
| Nisin | 1,200 g |
| Cetylstearylalkohol | 20,000 g |
| 2-Octyldodecanol | 20,000 g |
| Kaolin | 200,200 g |
| Talkum | 200,200 g |
| Aerosil | 48,200 g |
| Parfüm | nach Belieben |
| Reisstärke | auf 1 000,000 g |

### Beispiel 10

| | |
|---|---|
| Epidermin | 1,000 g |
| Cetylstearylalkohol | 20,000 g |
| 2-Octyldodecanol | 20,000 g |
| Kaolin | 200,200 g |
| Talkum | 200,200 g |
| Aerosil | 48,200 g |
| Parfüm | nach Belieben |
| Reisstärke | auf 1 000,000 g |

### Beispiel 11

### Waschgelkonzentrat

| | |
|---|---|
| Nisin | 9,000 g |
| Cocoamidopropylbetain | 613,300 g |
| Tipa-Laurylethersulfat | 306,700 g |
| Kochsalz | nach Belieben |
| Parfüm, Farbstoff | nach Belieben |
| Citronensäure | 1,000 g |
| Glycerin | 10,000 g |
| Wasser | auf 1 000,00 g |

### Beispiel 12

### Waschgelkonzentrat

| | |
|---|---|
| Epidermin | 7,000 g |
| Cocoamidopropylbetain | 613,300 g |
| Tipa-Laurylethersulfat | 306,700 g |
| Kochsalz | nach Belieben |
| Parfüm, Farbstoff | nach Belieben |
| Citronensäure | 1,000 g |
| Glycerin | 10,000 g |
| Wasser | auf 1 000,00 g |

### Beispiel 13

### Puderspray I

| a) Suspensionsgrundlage | |
|---|---|
| Polymethylsiloxan (Cyclomethicone) | 72,000 g |
| Talkum | 24,000 g |
| Bentonitgel IPM | 3,000 g |
| Nisin | 1,000 g |

| b) fertiger Spray | |
|---|---|
| Suspensionsgrundlage I | 20,000 g |
| Propan/Butan | 80,000 g |

### Beispiel 14

### Puderspray II

| a) Suspensionsgrundlage | |
|---|---|
| Polymethylsiloxan (Cyclomethicone) | 72,000 g |
| Titandioxid | 24,000 g |
| Bentonitgel IPM | 3,000 g |
| Nisin | 1,000 g |

| b) fertiger Spray | |
|---|---|
| Suspensionsgrundlage I | 20,000 g |
| Propan/Butan | 80,000 g |

### Beispiel 15

### Puderspray III

| a) Suspensionsgrundlage | |
|---|---|
| Polymethylsiloxan (Cyclomethicone) | 72,000 g |
| Seidenpulver | 24,000 g |
| Bentonitgel IPM | 3,000 g |
| Nisin | 1,000 g |

| b) fertiger Spray | |
|---|---|
| Suspensionsgrundlage I | 20,000 g |
| Propan/Butan | 80,000 g |

Die Zusammensetzungen gemäß den Beispielen 1 - 15 wurden anhand des sogenannten "Sniff-Tests" beurteilt.

Es wurden erfindungsgemäße Zusammensetzungen gegen Placebos, also bis auf den Gehalt an Wirksubstanz identische Zusammensetzungen getestet.
Ein Kollektiv von 40 Probanden hatte die Auflage, jeweils eine Achselhöhle mit erfindungsgemäßer Zusammensetzung und die andere mit Placebo zu behandeln. Die Probanden trugen daraufhin drei Stunden lang ein Hemd mit Slipeinlagen unter den Achseln. Nach Ablauf dieser Zeit wurden die Slipeinlagen in separate Flaschen überführt. Der Geruch der Einlagen wurde von drei Prüfpersonen beurteilt. Der Versuch wurde als Doppelblindversuch durchgeführt, so daß weder die Probanden noch die Prüfpersonen wußten, welche Achsel mit welcher Zusammensetzung behandelt worden war.

Es stellte sich heraus, daß die wirkstoffhaltigen Zubereitungen in jeweils 38 von 40 Fällen sensorisch beurteilt besser wirkten als die entsprechenden Placebos. In 2 von 40 Fällen gaben die Prüfpersonen an, behandelte und unbehandelte Proben würden sich nicht voneinander unterscheiden.

## Patentansprüche

1. Verwendung eines wirksamen Gehaltes von Lantibiotika in kosmetischen Desodorantien, wobei die Lantibiotika sowohl einzeln als auch im Gemisch vorliegen können.

2. Verwendung nach Anspruch 1, gekennzeichnet dadurch, daß die Lantibiotika gewählt werden aus der Gruppe Nisin, Epidermin, Subtilin, Cinramycin, Duramycin, Ancovenin, Pep 5, Gallidermin.

3. Verwendung nach Anspruch 1 oder 2, dadurch gekennzeichnet, daß die Desodorantien einen Gehalt von 0,1 - 10 000 ppm an Lantibiotika, bezogen auf das Gesamtgewicht der Formulierungen, aufweisen.

4. Verwendung nach einem der Ansprüche 1 - 3, dadurch gekennzeichnet, daß die Desodorantien einen Gehalt von 0,1 - 750 ppm an Lantibiotika, bezogen auf das Gesamtgewicht der Formulierungen, aufweisen.

5. Verwendung nach einem der Ansprüche 1 - 4, dadurch gekennzeichnet, daß die Desodorantien Gehalt von 0,5 - 400 ppm an Lantibiotika, bezogen auf das Gesamtgewicht der Formulierung, aufweisen.

6. Verwendung nach einem der Ansprüche 1 - 5, dadurch gekennzeichnet, daß die Desodorantien im sauren Bereich, vorzugsweise in einem Bereich von 2,5 - 6,5, abgepuffert sind.

7. Verwendung nach einem der Ansprüche 1 - 6, dadurch gekennzeichnet, daß die Desodorantien in einem pH-Bereich von 3,5 - 4,8 abgepuffert sind.

8. Verwendung nach einem der Ansprüche 1 - 7, dadurch gekennzeichnet, daß die Desodorantien in Form von Deo-Sprays, Deo-Roll-ons, Deo-Pumpsprays, desodorierenden Tinkturen, desodorierenden Intimreinigungsmittel, desodorierenden Shampoos, desodorierenden Dusch- oder Badezubereitungen, desodorierenden Pudern, desodorierenden Pudersprays oder Deo-Stiften vorliegen.

## Claims

1. Use of an effective content of lantibiotics in cosmetic deodorants, it being possible for the lantibiotics to be present both individually and in a mixture.

2. Use according to Claim 1, characterized in that the lantibiotics are chosen from the group comprising nisin, epidermin, subtilin, cinramycin, duramycin, ancovenin, Pep 5 and gallidermin.

3. Use according to Claim 1 or 2, characterized in that the deodorants have a content of 0.1 - 10,000 ppm of lantibiotics, relative to the total weight of the formulations.

4. Use according to one of Claims 1 - 3, characterized in that the deodorants have a content of 0.1 - 750 ppm of lantibiotics, relative to the total weight of the formulations.

5. Use according to one of Claims 1 - 4, characterized in that the deodorants have [lacuna] content of 0.5 - 400 ppm of lantibiotics, relative to the total weight of the formulation.

6. Use according to one of Claims 1 - 5, characterized in that the deodorants are buffered in the acid range, preferably in a range from 2.5 - 6.5.

7. Use according to one of Claims 1 - 6, characterized in that the deodorants are buffered in a pH range from 3.5 - 4.8.

8. Use according to one of Claims 1 - 7, characterized in that the deodorants are present in the form of deodorant sprays, deodorant roll-ons, deodorant pump sprays, deodorizing tinctures, deodorizing intimate cleansers, deodorizing shampoos, deodorizing shower or bath preparations, deodorizing powders, deodorizing powder sprays or deodorant sticks.

## Revendications

1. Utilisation d'une teneur efficace de lantibiotiques dans des déodorants cosmétiques, les lantibiotiques pouvant être présents non seulement individuellement, mais aussi sous forme de mélange.

2. Utilisation selon la revendication 1, caractérisée en ce que les lantibiotiques sont choisis parmi le groupe nisine, épidermine, subtiline, cinramycine, duramycine, ancovénine, pep 5, gallidermine.

3. Utilisation selon la revendication 1 ou 2, caractérisée en ce que les déodorants présentent une teneur de 0,1 - 10 000 ppm en lantibiotiques, par rapport au poids total des formulations.

4. Utilisation selon l'une quelconque des revendications 1 - 3, caractérisée en ce que les déodorants présentent une teneur de 0,1 - 750 ppm en lantibiotiques, par rapport au poids total des formulations.

5. Utilisation selon l'une quelconque des revendications 1 - 4, caractérisée en ce que les déodorants présentent une teneur de 0,5 - 400 ppm en lantibiotiques, par rapport au poids total de la formulation.

6. Utilisation selon l'une quelconque des revendications 1 - 5, caractérisée en ce que les déodorants sont tamponnés dans le domaine acide, de préférence dans un domaine de 2,5 - 6,5.

7. Utilisation selon l'une quelconque des revendications 1 - 6, caractérisée en ce que les déodorants sont tamponnés dans un domaine de pH de 3,5 - 4,8.

8. Utilisation selon l'une quelconque des revendications 1 - 7, caractérisée en ce que les déodorants sont disponibles sous la forme d'aérosols déodorants, de formulations déodorantes pour application au rouleau, d'aérosols déodorants pour pompage, de teintures déodorantes, d'agents déodorants de nettoyage intime, de shampoings déodorants, de préparations déodorantes pour le bain ou la douche, de poudres déodorantes, d'aérosols poudreux déodorants ou de bâtons déodorants.
